# EUROPEAN PATENT APPLICATION

(11) **EP 3 954 321 A1**
(43) Date of publication of application: **16.02.2022**
(21) Application number: 20191189.8
(22) Date of filing: 14.08.2020
(51) Int. Cl.: A61C 7/08, A61F 5/56, A63B 71/08

(54) **PROCESS FOR FABRICATING A DENTAL APPLIANCE AND NON-THERAPEUTICAL USE OF A DENTAL APPLIANCE**

(71) Applicant: Bottmedical AG, 4057 Basel (CH)
(72) Inventor: The designation of the inventor has not yet been filed
(74) Representative: Mertzlufft-Paufler, Cornelius

(57) **Abstract**

For improving the functionality and bio-compatibility of dental appliances (2), a novel process is proposed for making a thermoplastic functional foil (1), from which such dental appliances (2) may be formed by thermoforming the functional foil (1). A carrier liquid (9) containing an organic and preferably bio-based polymer (6) is enriched with an agent and applied onto a solid thermoplastic core foil (13). After evaporating of a solvent contained in the carrier liquid (9), a uniform and highly homogenous functional coating (5) is obtained on the core foil (13). After thermoforming of the foil (1), the dental appliance (2) thus features an outer protective coating (5) offering enhanced functionality (c.f. Figure 1).

## Description

The present disclosure relates to a process for making a thermoplastic functional foil comprising a core of thermoplastic material and a thermoplastic coating at least partially covering the core.

This foil is particularly suited for intra-oral use as a dental appliance to be worn inside the oral cavity on the teeth, since such an appliance may be easily formed from such a foil. Hence the disclosure also concerns a dental appliance fabricated, in particular formed, most preferably thermoformed from such a functional foil. The dental appliance may be used as an orthodontic appliance or a protective appliance for protection of teeth and/or gingiva of the user of the appliance.

Finally the disclosure concerns a novel non-therapeutic use-case, in which a dental appliance, as described before, is worn by a user for protecting his teeth and/or gingiva, in particular from cigarette smoke and/or from resulting gingivitis and/or parodontitis; moreover, by wearing the appliance on the teeth, the formation of plaque can be reduced and thus the risk to develop caries.

In the past, intraoral orthodontic devices have been used in orthodontic therapy for realigning teeth. In this specific application case, the devices are typically designed as a dental splint, which is applied to teeth over several hours. The dental splint exerts forces on the individual teeth of the patient, which results in a realignment of the teeth in directions of desired teeth positions. For this purpose, typically a number of dental splints, which vary slightly from each other in shape, are worn by the patient consecutively to realign the teeth step-wise.

Further known applications of such intraoral orthodontic devices, in particular dental splints, are bleaching of teeth using bleaching pastes which are applied to the teeth by the device, or use of such devices for treating snoring overnight.

Intraoral appliances such as orthodontic devices are also used for avoiding clenching and grinding. Chronic teeth clenching and teeth grinding can cause overuse of the muscles controlling the lower jaw, leading to pain from those muscles. The load on the joint itself can also cause changes inside the joint, leading to pain and limited opening of the mouth.

In all of these applications, providing a high wearing comfort of the device is of central importance for a high acceptance of the treatment by the patient and hence overall success of the therapy. But also when a dental appliance is worn purely for protection of teeth, a high wearing comfort is an important quality.

In this context, the invention aims at further enhancing the functionality and biocompatibility of such dental appliances and - in relation to this fundamental aim - at providing an efficient method for fabricating such devices.

In accordance with the present invention, a process is provided according to claim 1, which solves the afore-mentioned problem. In particular, the invention proposes a process for making a thermoplastic functional foil as described at the beginning featuring a thermoplastic core and a thermoplastic coating at least partially, but preferably fully, covering the core. This process comprises the following steps: providing a carrier liquid comprising an organic polymer; applying the carrier liquid as the coating onto the core.

In other words, the invention suggest to apply the coating in the form of liquid coating onto a solid core, which may have the form of a core foil, in particular onto both sides of such a core foil, and to solidify the liquid coating afterwards on the core to form a solid thermoplastic coating covering the core.

Through this process, a highly uniform and homogenous coating of the core can be achieved, and both the core and the coating can be thermoformed together in a later process step, as will be explained in greater detail below. Another great advantage of this liquid coating approach is that the coating can provide new functionalities to the foil and dental appliances formed from the foil. In particular, as will be explained in greater detail below, an agent may be mixed into the carrier liquid forming the coating prior to solidifying the coating on the core.

The coating may be formed, alternatively, using a hot melt extrusion process and providing the carrier liquid as a carrier melt. Such a carrier melt may also be applied onto a solid core to form a functional coating.

As will become clear below, the coating may form a thermoplastic cap layer of a dental appliance fabricated from the foil. For particular applications, for example when antimicrobial protection is to be provided, it can be beneficial if the cap layer fully covers the core of the device, and hence the thermoplastic coating may fully cover the core of the functional foil.

According to the invention, there exist further advantageous embodiments solving the aforementioned problems, which are described in the sub-claims and in the following.

For example, one embodiment of the process suggests to add an agent to the carrier liquid prior to forming the coating. Preferably, the agent may be added to the carrier liquid in the form of an agent liquid, as many agents can be obtained already in liquid form. Such an agent may be in particular an agent that is releasable from the coating in aqueous environments. This has the advantage that such an agent can be released from the coating into an oral cavity of a user that is wearing a dental appliance formed from the functional foil.

Another highly preferred embodiment of the process suggests that the agent is derived from a bio-based material. The term "bio-based material" may be understood here in the sense that a bio-based material may comprise a non-fossil carbon content containing a detectable portion of the carbon isotope ¹⁴C. Preferably, the non-fossil carbon content may constitute at least 20%, preferably at least 35%, most preferably more than 50% of the bio-based material. In other words, about 80% to 65% but preferably much less of the bio-based material can be based on other organic material, in particular polymers, derived from fossil sources such as crude oil.

As will be explained later, such a bio-based material may also be used as the material for the coating and/or such a bio-based material may fully cover a core of a dental appliance fabricated from said functional foil, with the core not being made from a bio-based material.

¹⁴C is a radioactive isotope produced in the earth's atmosphere at a concentration of about 1.25.10⁻¹² = 1250 ppq (ppq = part per quadrillion = 10⁻¹⁵) and incorporated into plants by photosynthesis. If a plant dies, it stops exchanging carbon with its environment, and thereafter the amount of ¹⁴C it contains begins to decrease as the ¹⁴C undergoes radioactive decay. The older a sample containing such biomaterial is, the less ¹⁴C remains to be detected. Hence by detecting the presence of ¹⁴C, material containing non-fossil carbon content can be differentiated from material derived from fossil sources. The presence of the ¹⁴C in a material is thus a direct proof that the material is obtained, at least in part, from non-fossil renewable biological sources. This is because organic polymers derived from fossil oil for example do not show any ¹⁴C any more, since the half-time of radioactive ¹⁴C is less than 6.000 years.

"Detectable portion" may be understood here in that the fraction of the ¹⁴C among all C-atoms of the non-fossil carbon content of the bio-based material may be at least 1 ppq, preferably at least 10 ppq or even at least 100 ppq.

The bio-based material may be further characterized in that it comprises a bio-based content of at least 40%, preferably of at least 50%, and wherein said bio-based content includes the non-fossil carbon content containing ¹⁴C described above as well as nitrogen, oxygen and hydrogen bound to the non-fossil carbon content.

In other words, at least part of the coating may be based on one or more bio-based materials. Preferably, however, the coating may be fabricated exclusively from bio-based materials.

Most preferably, the agent may be derived from an essential oil extracted directly from non-fossil plants. Such embodiments ensure that the bio-based agent is not harmful to the body of the user of the dental appliance. Moreover, with such an approach, a myriad of different functionalities such as adding flavor or antimicrobial protection may be achieved by relying on natural products that do not lead to harmful contaminations inside the oral cavity.

According to another preferred embodiment, the agent may comprise at least one of the following substances: an essential oil, an extract of an essential oil, or a derivative such as cinnamaldehyde, lime, thymol, eugenol, linalool, carvacrol, nutmeg, pimenta berry, rosemary, petitgrain, coffee, anise.

Further extracts/derivatives obtained from bio-based materials, in particular through extraction from a natural essential oil, that may be used as an agent to be embedded in the coating, are + a, β-pinene, myristicin, methyl eugenol, menthol, terpinene, p-cymene, linalool, myrcene, pinene, β-ocimene, geranial, sabinene, neral, citronellol, linolenic acid, oleic, stearic, himachalene, bisabolene or trans-cinnamaldehyde.

A particular advantage of using lime and cinnamaldehyde as agents is that they are not only highly effective antimicrobial agents but also provide a softening and plasticizing effect to the thermoplastic coating.

To further enhance the safety and compliance of dental appliances formed from the functional foil, it is highly preferable, if the organic polymer contained in the carrier liquid is a bio-based material or at least derived from a bio-based material.

A highly preferred embodiments suggests to use cellulose acetate butyrate (CAB) as the organic polymer. In this case, it is highly preferable for good mechanical properties of the thermoplastic functional foil (and dental appliances formed from the foil), if a butyryl content of the CAB is between 15 to 60% by weight.

By providing such features, it can be achieved that the agent embedded into the coating is releasable from the coating, in particular in aqueous environments, for example in the oral cavity. Moreover, it can be achieved by adequate choice of the agent, for example by using cinnamaldehyde as the agent, that the coating provides antimicrobial protection.

To improve the malleability and adaptiveness of the foil during thermoforming, it is of advantage if a glass transition temperature T_{g,coating} of the coating, and/or in particular a glass transition temperature of the organic polymer T_{g,polymer}, is/are in the range of 80-165°C. This is particularly true when using CAB as the organic polymer.

A suitable corresponding melting range of the coating, and in particular said organic polymer, may be from 120-200°C.

Another important factor for achieving satisfying results when thermoforming the functional foil is the number average molecular weight of the organic polymer used for the coating. For optimum results, the number average molecular weight may be more than 12.000 g/mol, preferably more than 20.000 g/mol, and most preferably more than 30.000 g/mol. Such numbers are particularly suited if CAB is chosen as the organic polymer.

Another highly advantageous embodiment of the process introduced at the beginning proposes that the carrier liquid is a carrier solution, i.e. the carrier liquid may be a solution of a solvent and the organic polymer. In this case, it if preferable if the carrier solution is obtained by dissolving the organic polymer in a solvent. This has the advantage that the coating may be solidified on the core by evaporating the solvent from the coating. This approach is of advantage because the solvent can be used to modify the surface of the core, as will be explained later. As already said, the core may actually be a core foil and hence, the carrier solution may be applied to both sides of the core foil uniformly.

For achieving a uniform coating thickness, the coating can be formed by a physical coating process such as spin coating or blade-based coating, or by spray coating or by roll-to-roll coating. Of course there is a myriad of different processes are available for performing roll-to-roll coating (e.g. air knife coating, gap coating, immersion dip coating, roller coating, etc.) or spray coating (spray painting, thermal spraying, plasma spraying etc.), as examples.

As already mentioned, the solvent of the carrier solution can be used to modify the surface of the core. In fact, the solvent can soften the core's surface by reducing the interaction of molecular chains of the material of the core. As a result, after treating the core with the carrier solution, the core and the coating (or to be more precise, the organic polymer of the coating) can interlock on a nanometer scale, resulting in improved adhesion of the coating on the core. After application of the coating in liquid form onto the core and interaction of the solvent with the core, the solvent may be simply evaporated thus forming a final solid coating.

Such as softening of the core can be achieved, for example, when using acetone as the solvent and PETG as the core material, but there exist other combinations of core materials and suitable solvents, which achieve the same softening effect, as is readily apparent in the literature. The invention suggests to use this effect for enhancing the adhesion of the coating on the core of a functional foil intended for fabricating a dental appliance.

Another approach for improving the mechanical and/or chemical interaction of the coating and the core is to safeguard that a glass transition temperature T_{g,core} of the core and a glass transition temperature T_{g,coating} of the coating differ by less than 80°C, preferably by less than 60°C. Such a material choice will result in enough mobility of the molecular chains of both core and coating such that efficient thermal interlocking can be achieved through thermal fusion (sometimes referred to as "thermal welding") between core and coating during the final thermoforming of the functional foil.

Moreover, in such a case, it can be of advantage, if the glass transition temperature of the coating T_{g,coating} is higher than the glass transition temperature of the core T_{g,core}. Such a material choice will result in a sufficient mechanical stability during thermoforming of the device, as the organic polymer can provide some stability to the foil. For example, PETG, which is a useful material for the core, can have a glass transition temperature of 80°C, and a melting temperature as high as 210°C. Such a core can be combined with a coating based on cellulose acetate butyrate (CAB), and such a CAB-coating may feature a glass transition temperature T_{g,coating} of 120°C or above, but at the same time a relatively low melting temperature T_{m,coating} of 160°C.

Additionally, either the coating should reach its melting temperature T_{m,coating} before the core reaches its melting temperature T_{m,core} or the core should reach its melting temperature before the coating reaches its own. This may be achieved - in particular when considering thermoforming - by safeguarding that the melting temperature of the core T_{m,core} the melting temperature of the coating T_{m,coating} differ by at least 20°C, preferable at least 40°C, most preferably at least 60°C. As a result, a compound material may be produced at the interface between core and coating during thermoforming of the functional foil, because when for example the coating reaches its melting temperature, the core may still be in the glass transition phase/temperature range (or vice versa). This compound material will thus comprise chains from the organic polymer of the thermoplastic coating and parts of the material used for the thermoplastic core.

Preferable for easy fabrication, however, will be a design, in which the melting temperature of the core T_{m,core} is at least 20°C, preferable at least 40°C, most preferably at least 60°C, higher than the melting temperature of the coating T_{m,coating}. In such a design, the coating will become molten while the core material is still solid or still in the glass transition stage.

The solvent used for the carrier solution may comprise at least one of acetone, methyl acetate, ethyl acetate, methylethyl ketone, isopropyl acetate, butyl acetate, ethyl lactate, cyclohexane, diacetone alcohol, butyl lactate or suitable mixtures of these solvents.

Another variant of the process is defined in that the agent itself may be a liquid. In this case, the agent in liquid form (agent liquid) may be simply mixed with the carrier liquid.

Alternatively, the agent may be added to the carrier liquid by dissolving or emulsifying the agent first in an aqueous or oil-based solution and mixing this solution (agent liquid) with the carrier liquid. In such a case, it is preferable for a homogenous distribution of the agent within the final coating, if the solution containing the agent is mixed with the carrier liquid, for example by a stirrer, to form a homogenous carrier solution, prior to forming the coating.

Concerning the ratio between the agent and the organic polymer in the final coating, this ratio may be typically between 0.01/99.99 and 30/70 by weight, for example 2-3g of agent and 7g of organic polymer. For most applications, it will be preferable if the ratio between the agent and the organic polymer is between 0.1/99.9 and 20/80 by weight.

A particular suitable embodiment of the process can be obtained, if the core of the functional foil is of Polyethylenterephthalat (PET). For improving the mechanical properties of the functional foil, in particular for reducing the tendency for crack formation, glykol modified Polyethylenterephthalat(PETG) may be chosen as the material for the core. In these cases, a highly suitable solvent for achieving the softening effect described above is acetone.

The process may be further modified in that a natural softening agent, preferably triacetin or polycaprolactonetriol (PCL-T), is added as a plasticizer to the carrier liquid prior to forming the coating. This may be done, preferably, at a volume ratio of less than 40%, preferably of less than 20%, most preferably of less than 10 %.

Finally, the process explained so far may be continued by forming a dental appliance from the functional foil by thermoforming the foil. Importantly, this thermoforming may be done after application of the coating onto the core, as both the core and the coating may be thermoplastic. As a result, after thermoforming of the functional foil, the organic polymer may form a conformal functional coating, preferably fully covering the core of the foil, which then constitutes the core of the dental appliance. Moreover, an agent previously embedded in the coating may then provide antimicrobial protection or regenerative functionality for teeth and gingiva or a flavor or simply a nice color to the dental appliance.

An important aspect with regard to the final thermoforming is the thermal stability of the agent. In order to safeguard the proper functionality of the final device, a decomposition temperature of the agent T_{d,agent} should be at least 10°C above the melting temperature T_{m,coating} of the coating (the decomposition temperature T_{d,agent} may thus lie up to 60°C above the glass transition temperature T_{g,coating} of the coating) .

Following the concept outlined above, the afore-mentioned problem may thus be solved also by a dental appliance according to the invention. As described at the beginning, this dental appliance may be an orthodontic appliance or a protective appliance for protection of teeth and/or gingiva, and it may be formed, in particular thermoformed, from a functional foil fabricated with a process as described herein or as described by one of the claims directed towards such a process. In such a dental appliance, the agent described above with respect to the functional foil may be releasable from the dental appliance during intra-oral use. According to a particular approach intended for using the dental appliance purely for protection of teeth and gingiva, the dental appliance may consist entirely of the functional foil, which may have a low thickness of less than 400 µm, preferably of less than 300 µm, most preferably of less than 200 µm.

Finally, following the above concept of making a functional thermoplastic foil offering increased functionality, a novel use case of a dental appliance is suggested, to benefit from the increased functionality. In other words, a non-therapeutic use of a dental appliance is proposed for protecting teeth and/or gingiva of a user wearing the dental appliance on his/her teeth. This protection can be in particular from cigarette smoke and/or resulting gingivitis and/or parodontitis and the dental appliance may be fabricated as previously described. The wearing of the protective dental appliance on the teeth can also provide protection from plaque formation and thus reduce the risk to develop caries. This is particularly true when using lime and cinnamaldehyde as an agent embedded in a coating of the dental appliance.

But apart from the approach of fabricating such a dental appliance, the use case is characterized in that during use, the dental appliance covers both teeth and parts of the gingiva adjoining to the teeth, and in that the dental appliance features a functional coating offering antimicrobial protection. This protection may be provided, as has been explained above, in particular through a releasable antimicrobial agent and this agent may be embedded in the functional coating, in particular as described previously. Most preferably for this use case is a situation, in which the coating is formed from a bio-based material and/or the agent is also derived from a bio-based material. This further enhances the protection and safety when wearing the appliance.

Moreover, in such a use case, it is preferable if the dental appliance is formed with cavities matching the natural positions of the teeth of a user such that the appliance does not exert forces onto the teeth. This greatly improves the wearing comfort, which is important, if the user wants to profit from long-time protection by wearing the dental appliance over hours. For the same reason, it is regarded highly preferable, if the thermoplastic functional foil shows a maximum thickness of less than 700 µm, preferably of less than 500 µm, most preferably of less than 300 µm (after the final thermoforming process). This is because, in particular due to the liquid coating approach, the thickness of the functional coating may be less than 100 µm or even less than 50 µm, while the coating may be still providing sufficient antimicrobial protection, due to a high concentration of the agent inside the coating. In particular, such a dental appliance can be comfortably worn by a user while the user is smoking or sleeping.

Preferred embodiments of the present invention shall now be described in more detail, although the present invention is not limited to these embodiments: for those skilled in the art it is obvious that further embodiments of the present invention may be obtained by combining features of one or more of the patent claims with each other and/or with one or more features of an embodiment described or illustrated herein.

With reference to the accompanying drawings, where features with corresponding technical function are referenced with same numerals even when these features differ in shape or design:
- Fig. 1: is a schematic overview of a fabrication process according to the invention,
- Fig. 2: illustrates the definition of non-fossil carbon content and bio-based content used herein to describe bio-based materials.

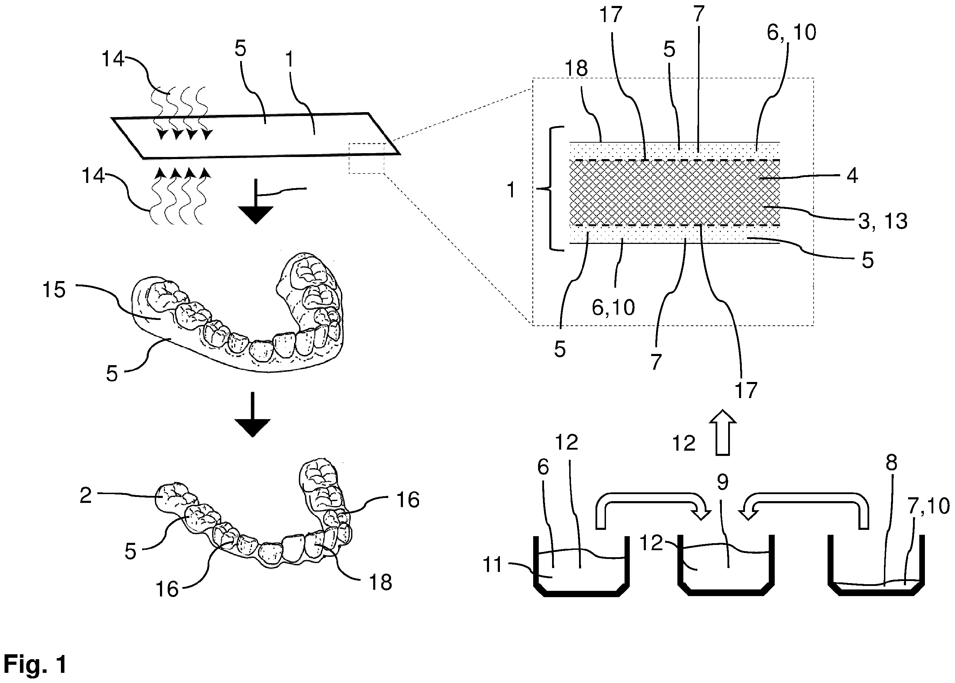

Figure 1 provides an overview of a fabrication process according to the invention: An organic polymer 6, namely cellulose acetate butyrate (CAB), is dissolved in a solvent 12, namely acetone, to form a carrier solution 11. The carrier solution 11 is next mixed with an agent liquid 8 containing cinnamaldehyde, which is a bio-based antimicrobial agent 7, to form a carrier liquid 9, in which the agent 7 and the CAB are homogenously mixed. This carrier liquid 9 is then applied by dip coating onto a core foil 13 of a thermoplastic material 4.

After evaporation of the solvent 12, a solid thermoplastic coating 5 is obtained on both sides of the core-foil 13, which thus completely covers the core 3 of the resulting functional foil 1 (c.f. the cross-section visible in Figure 1).

The foil 1, in fact, constitutes a multi-layer structure with a core 3 made from glykol modified Polyethylenterephthalat (PETG) with a thickness of 200 µm and top and bottom functional coatings 5 which each have a thickness (after solidification) of less than 20 µm, thus resulting in a total thickness of the thermoplastic functional foil 1 of less than 250 µm.

As a next step, using a 3D-model of an oral cavity of a patient, a pre-form 15 is formed from the functional foil 1 by thermoforming, applying heat 14 from both sides to the foil 1 and thus distorting the core 3 and the coatings 5 together in one step. In other words, the coating 5 is already firmly linked to the core 3 prior to thermoforming. The underlying reason is that the acetone 12 modifies the surface of the PETG core 3 leading to a softening of the PETG surface such that the CAB molecules 6 can interlock on a nanometer scale at the interface 17 (cf. Figure 1) with the PETG of the core 3. As a result, there is already a high adhesion of the coating 5 on the core 3 prior to thermoforming.

As the glass transition temperatures T_{g,core} of the PETG-core 3 and T_{g,coating} of the coating differ by less than 60°C, during thermoforming the adhesion of the coating 5 on the core foil 13 is further improved, as a thermal interlocking is achieved through thermal fusion of the CAB-coating 5 with the PETG.

Finally the pre-form 15 is cut such that the resulting dental appliance 2 visible on the bottom left of Figure 1 covers both teeth and adjoining parts of the gingiva. As visible, the dental appliance 2 further features multiple cavities 16 designed for taking up single teeth and which are matching the natural positions of the teeth of the user. Thus, the user experiences no forces on his teeth when wearing the dental appliance 2 on his teeth. As the appliance 2 is very thin, highly conformable and offers a soft and highly deformable functional coating 5, which, due to the CAB used, even softens when getting into contact with saliva and offers antimicrobial protection due to the embedded cinnamaldehyde, the appliance 2 is ideally suited to be worn overnight or while smoking, even for hours. In such non-therapeutic use-cases, the user can benefit from the protection provided by the appliance 2 against cigarette smoke, as the appliance 2 is impermeable to smoke, but also from gingivitis and parodontitis, due to the antimicrobial effect that is produced when the cinnamaldehyde 7 is slowly released from the coating 5 into the oral cavity of the user.

The CAB used for the coating 5 encapsulating the core 3 as illustrated on the right of Figure 1 is in fact a bio-based material 10, as it is produced by blending organic materials derived from fossil sources with organic material, in particular cellulose, derived from non-fossil natural sources such as plants. Therefore, the complete outer surface 18 of the appliance 2 is formed from bio-based materials 10. Any mechanical abrasion from this surface 18 will thus not be harmful for the user wearing the appliance 2 in his mouth.

Likewise, the agent 7 embedded in the coating 5 can be derived from natural sources such as essential oils extracted directly from non-fossil plants. This allows use of agents such as lime, thymol, eugenol, linalool, carvacrol, nutmeg, pimenta berry, rosemary, petitgrain, coffee, anise, to name a few. Hence the appliance 2 can deliver both flavor and antimicrobial protection and still be highly biocompatible and non-harmful to wear.

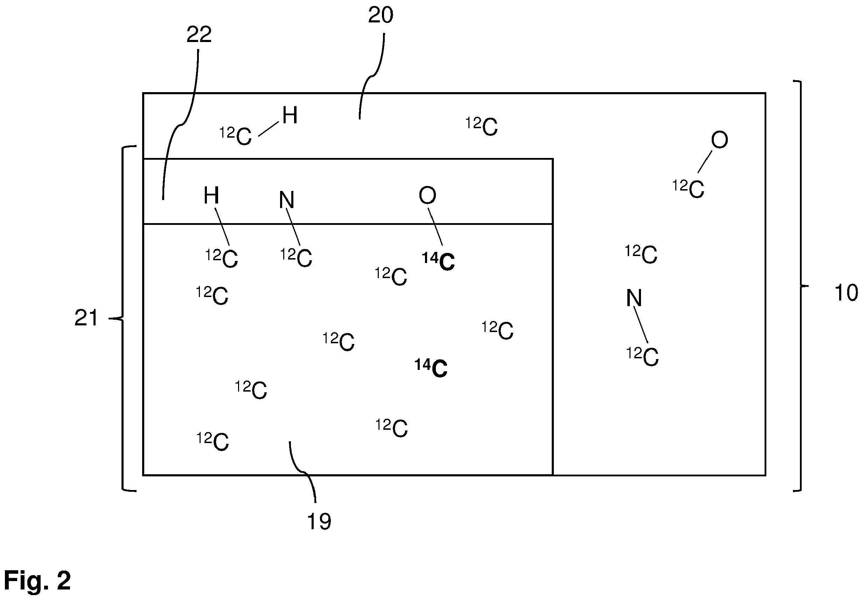

As Figure 2 illustrates schematically, "bio-based material" as understood herein may mean that the organic polymer 6 as well as the agent 7 used for the coating 5 may contain a significant portion of non-fossil carbon content 19, for example at least 40%. This non-fossil carbon content 19 is characterized in that it contains the carbon isotope ¹⁴C in a detectable fraction (e.g. more than 10 ppq or even more than 100 ppq). There may also be a fossil carbon content 20 (cf. Figure 4), which is based on fossil sources and containing the isotope ¹²C but no detectable fraction of ¹⁴C any more, as the ¹⁴C has been diminished by radioactive decay over thousands of years. As illustrated in Figure 4, there can be defined a further quantity namely the so-called bio-based content 21. This fraction of the material 5 comprises the non-fossil carbon content 19 as well as all hydrogen H-, oxygen O-, and nitrogen N-atoms 22 bound to the non-fossil carbon content 19.

In summary, for improving the functionality and biocompatibility of dental appliances 2, a novel process is proposed for making a thermoplastic functional foil 1, from which such dental appliances 2 may be formed by thermoforming the functional foil 1. A carrier liquid 9 containing an organic and preferably bio-based polymer 6 is enriched with an agent 7 and applied onto a solid thermoplastic core foil 13. After evaporating of a solvent 12 contained in the carrier liquid 9, a uniform and highly homogenous functional coating 5 is obtained on the core foil 13. After thermoforming of the foil 1, the dental appliance 2 thus features an outer protective coating 5 offering enhanced functionality (c.f. Figure 1).

### List of reference numerals

- 1: thermoplastic functional foil
- 2: dental appliance
- 3: core
- 4: thermoplastic material
- 5: thermoplastic coating
- 6: organic polymer
- 7: agent
- 8: agent liquid
- 9: carrier liquid
- 10: bio-based material
- 11: carrier solution
- 12: solvent
- 13: core foil
- 14: heat applied
- 15: pre-form
- 16: cavities (for taking up single teeth)
- 17: interface
- 18: outer surface
- 19: non-fossil carbon content (containing ¹⁴C)
- 20: carbon content based on fossil sources (containing no detectable fraction of ¹⁴C any more)
- 21: bio based content (i.e. non-fossil carbon content plus hydrogen, nitrogen, and oxygen bound to this content)
- 22: hydrogen, nitrogen, and oxygen bound to non-fossil carbon

## Claims

1. **Process** for making a thermoplastic functional foil (1), in particular for intra-oral use as a dental appliance (2), wherein the functional foil (1) comprises a core (3) of thermoplastic material (4) and a thermoplastic coating (5) at least partially covering the core (3), and wherein the process comprises the following steps:
- providing a carrier liquid (9) comprising an organic polymer (6);
- applying the carrier liquid (9) as the coating (5) onto the core (3);

2. Process according to claim 1,
- wherein an agent (7), in particular an agent (7) releasable from the coating (5) in aqueous environment, is added to the carrier liquid (9) prior to forming the coating (5), preferably in the form of an agent liquid (8),
- preferably wherein the agent (7) is derived from a bio-based material (10),
- most preferably from an essential oil extracted directly from non-fossil plants.

3. Process according to claim 2,
- wherein the agent (7) comprises at least one of the following substances: an essential oil, an extract of an essential oil, or a derivative such as cinnamaldehyde, lime, thymol, eugenol, linalool, carvacrol, nutmeg, pimenta berry, rosemary, petitgrain, coffee, or anise.

4. Process according to any one of the preceding claims,
- wherein the organic polymer (6) is a bio-based material (10),
- preferably cellulose acetate butyrate (CAB),
- most preferably wherein a butyryl content of the CAB is between 15 to 60% by weight and/or
- in particular such that the agent (7) is releasable from the coating (5) and/or
- wherein the coating (5) provides antimicrobial protection.

5. Process according to any one of the preceding claims,
- wherein a glass transition temperature T_{g,coating} of the coating (5) is in the range of 80-165°C,
- preferably wherein a decomposition temperature T_{d,agent} of the agent (7) is at least 10°C above a melting temperature T_{m,coating} of the coating (5), and/or
- wherein a corresponding melting range of the coating (5), in particular of said organic polymer (6), is in the range of 120-200°C,
and/or
- wherein the organic polymer (6), in particular the CAB, used for the coating (5) has a number average molecular weight of more than 12.000 g/mol, preferably of more than 20.000 g/mol.

6. Process according to any one of the preceding claims,
- wherein the carrier liquid (9) is a carrier solution (11),
- preferably wherein the carrier solution (11) is obtained by dissolving the organic polymer (6) in a solvent (12),
- most preferably wherein the coating (5) is solidified on the core (3) by evaporating the solvent (12) from the coating (5) and/or
- wherein the core (3) is a core foil (13).

7. Process according to any one of the preceding claims,
- wherein the coating (5) is formed by a physical coating process such as spin coating or blade-based coating, or by spray coating or by roll-to-roll coating.

8. Process according to any one of the preceding claims,
- wherein a glass transition temperature T_{g,core} of the core (3) and a glass transition temperature T_{g,coating} of the coating (5) differ by less than 80°C, preferably by less than 60°C, in particular wherein T_{g,coating} is higher than T_{g, core},
- most preferably wherein a melting temperature T_{m,core} of the core (3) and a melting temperature T_{m,coating} of the coating (5) differ by at least 20°C, preferably by at least 40°C, most preferably wherein the melting temperature T_{m,core} of the core (3) is higher than the melting temperature T_{m,coating} of the coating (5).

9. Process according to any one of the claims 6 to 8,
- wherein the solvent (12) modifies the surface of the core (3), in particular by reducing the interaction of molecular chains of the material of the core (3), such that the core (3) and the coating (5) can interlock on a nanometer scale, resulting in improved adhesion of the coating (5) on the core (3), and/or
- wherein the solvent (12) comprises at least one of acetone, methyl acetate, ethyl acetate, methylethyl ketone, isopropyl acetate, butyl acetate, ethyl lactate, cyclohexane, diacetone alcohol, butyl lactate or suitable mixtures of these solvents.

10. Process according to any one of the claims 2 to 9,
- wherein the agent (7) itself is a liquid or wherein the agent (7) is added to the carrier liquid (9) by dissolving or emulsifying the agent (7) in an aqueous or oil-based solution and mixing the solution with the carrier liquid (9),
- preferably wherein the solution containing the agent (7) is mixed with the carrier liquid (9), e.g. by a stirrer, to form a homogenous carrier solution (11), prior to forming the coating (5).

11. Process according to any one of the preceding claims,
- wherein a ratio between the agent (7) and the organic polymer (6) is between 0.01/99.99 and 30/70 by weight.

12. Process according to any one of the preceding claims,
- wherein the core (3) is made from Polyethylenterephthalat (PET),
- preferably from glykol modified Polyethylenterephthalat (PETG),
- most preferably wherein the solvent (12) is acetone.

13. Process according to any one of the preceding claims,
- wherein a dental appliance (2) is formed from the functional foil (1) by thermoforming the foil (1) after application of the coating (5) to the core (3),
- in particular such that after thermoforming, the organic polymer (6) forms a conformal functional coating (5), preferably fully covering the core (3),
- in particular wherein the agent (7) embedded in the coating (5) provides antimicrobial or regenerative functionality for teeth and gingiva or a flavor to the dental appliance (2).

14. **Dental appliance** (2), such as an orthodontic appliance or a protective appliance for protection of teeth and/or gingiva, formed, in particular thermoformed, from a functional foil (1) fabricated with a process according to any one of the preceding claims,
- in particular wherein the agent (7) is releasable from the dental appliance (2) during intra-oral use,
- preferably wherein the dental appliance (2) consists entirely of the foil (1).

15. **Non-therapeutic use of a dental appliance** (2), in particular according to the previous claim, for protecting teeth and/or gingiva, in particular from cigarette smoke and/or resulting gingivitis and/or parodontitis, **characterized in that**
- during use, the dental appliance (2) covers both teeth and parts of the gingiva adjoining to the teeth, and
- wherein the dental appliance (2) features a functional coating (1) offering antimicrobial protection, in particular through a releasable antimicrobial agent (7),
- preferably wherein the dental appliance (2) is formed with cavities matching the natural positions of the teeth of a user such that the dental appliance (2) does not exert forces onto the teeth,
- preferably wherein the thermoplastic functional foil (1) shows a maximum thickness of less than 700 µm, preferably of less than 500 µm, most preferably of less than 300 µm,
- in particular wherein the dental appliance (2) is worn by a user while the user is smoking or sleeping.
